(19) Europäisches Patentamt
European Patent Office
Office européen des brevets

(11) Veröffentlichungsnummer: **0 103 143**
**B1**

(12) # EUROPÄISCHE PATENTSCHRIFT

(45) Veröffentlichungstag der Patentschrift: **08.07.87**

(21) Anmeldenummer: **83107685.6**

(22) Anmeldetag: **04.08.83**

(51) Int. Cl.⁴: **C 07 D307/38**, C 07 D307/52, C 07 D231/12, C 07 D233/54, C 07 D249/08, C 07 D213/50, C 07 D213/70, C 07 D309/06, C 07 D285/12, C 07 D277/16, C 07 D261/08// C07D239/38, C07D277/74, C07D263/58, C07D209/14, C07D333/22, A01N43/10, A01N43/34, A01N43/653

(54) **Cyclohexan-1,3-dion-derivate, Verfahren zu ihrer Herstellung und ihre Verwendung zur Bekämpfung unerwünschten Pflanzenwuchses.**

(30) Priorität: **13.08.82 DE 3230087**

(43) Veröffentlichungstag der Anmeldung: **21.03.84 Patentblatt 84/12**

(45) Bekanntmachung des Hinweises auf die Patenterteilung: **08.07.87 Patentblatt 87/28**

(84) Benannte Vertragsstaaten: **DE FR GB IT**

(56) Entgegenhaltungen:
**EP-A- 0 070 370**
**EP-A- 0 071 707**
**DE-A- 2 439 104**
**CHEMICAL ABSTRACTS, Band 85, 1976, Seite 423, Nr. 5281g; COLUMBUS, OHIO, (U.S.)**
**CHEMICAL ABSTRACTS, Band 93, 1980, Seite 322, Nr. 39515s; COLUMBUS, OHIO, (US)**
**CHEMICAL ABSTRACTS, Band 94, 1981, Seite 94, Nr. 11639g; COLUMBUS, OHIO, (US)**

(73) Patentinhaber: **BASF Aktiengesellschaft**
**Carl-Bosch-Strasse 38**
**D-6700 Ludwigshafen (DE)**

(72) Erfinder: **Jahn, Dieter, Dr.**
**Burgunder Weg 8**
**D-6803 Edingen-Neckarhausen (DE)**
Erfinder: **Becker, Rainer, Dr.**
**Im Haseneck 22**
**D-6702 Bad Duerkheim (DE)**
Erfinder: **Goetz, Norbert, Dr.**
**Schoefferstrasse 25**
**D-6520 Worms 1 (DE)**
Erfinder: **Keil, Michael, Dr.**
**Limesstrasse 3**
**D-6700 Ludwigshafen (DE)**
Erfinder: **Wuerzer, Bruno, Dipl.-Landwirt., Dr.**
**Ruedigerstrasse 13**
**D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 103 143 B1

**Beschreibung**

Die Erfindung betrifft Cyclohexan-1,3-dion-derivate, Verfahren zu ihrer Herstellung, sowie Herbizide, die diese Verbindungen als Wirkstoff enthalten.

Es ist bekannt, Cyclohexan-1,3-dion-derivate zur Bekämpfung von unerwünschten Gräsern in breitblättrigen Kulturen anzuwenden (DE-OS-24 39 104, Chem. Abstr. Vol. 85 (1976) Nr. 5281g, Chem. Abstr. Vol. 94 (1981) Nr. 11639g). In den älteren Patentanmeldungen EP-A-71 707 und EP-A-70 370 werden ebenfalls Cyclohexan-1,3-dion-derivate mit herbizider Wirkung beschrieben.

Es wurde gefunden, daß Cyclohexan-1,3-dion-derivate der Formel

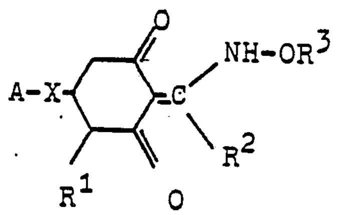

(I)

in der

A einen Pyridyl-, Tetrahydropyranyl- oder Dihydropyranylrest bedeutet, der gegebenenfalls durch $C_1$-$C_4$-Alkyl, $C_1$-$C_4$-Alkoxy oder Phenyl substituiert sein kann,

X eine Alkylenkette mit bis zu 5 C-Atomen oder eine Alkenylenkette mit bis zu 5 C-Atomen und bis zu 2 Doppelbindungen, die gegebenenfalls ein Schwefel- oder Sauerstoffatom oder eine Sulfinyl- oder Sulfonylgruppe enthalten und durch 2 Methylgruppen und Chlor substituiert sein können mit der Maßgabe, daß X keine reine gesättigte Kohlenstoffkette ist, wenn A ein nichtaromatischer heterocyclischer Rest mit maximal einer Doppelbindung und 1 Sauerstoffatom ist,

$R^1$ Wasserstoff, Methoxycarbonyl, Ethoxycarbonyl, Methyl oder Cyano,

$R^2$ Alkyl mit 1 bis 4 C-Atomen und

$R^3$ Alkyl mit 1 bis 3 C-Atomen, Alkenyl mit 3 oder 4 C-Atomen, Halogenalkenyl mit 3 oder 4 C-Atomen und 1 bis 3 Halogensubstituenten oder Propargyl bedeuten,

und Salze dieser Verbindungen gegen Gräser herbizid wirksam sind und sowohl breitblättrige Kulturpflanzen und monokotyle Kulturen, welche nicht zur Familie der Gräser (Gramineen) zählen, als auch überraschenderweise Getreide nicht oder nur wenig schädigen.

Die Verbindungen der Formel I können in mehreren Formen auftreten, die alle vom Patentanspruch umfaßt werden :

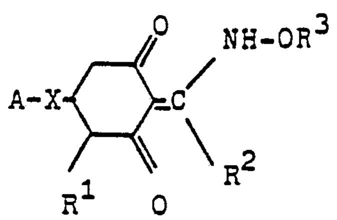

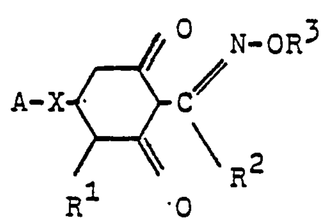

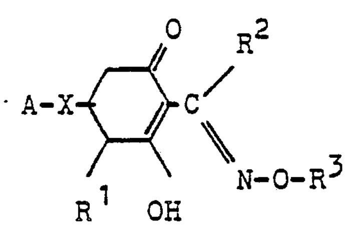

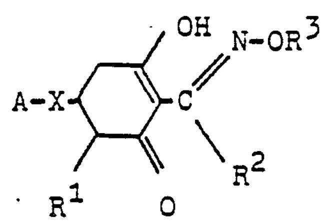

A in Formel I bedeutet beispielsweise einen gegebenenfalls durch Alkyl mit 1 bis 4 C-Atomen, Alkoxy mit 1 bis 4 C-Atomen oder Phenyl einfach oder mehrfach substituierten Pyridyl-, Tetrahydropyranyl-, Dihydropyranyl- oder 4-Methyl-tetrahydropyran-3-ylrest.

X in Formel I kann z. B. folgende Bedeutungen haben : $-CH_2-$, $-(CH_2)_2-$, $-CH(CH_3)-CH_2-$, $-CH_2-CH(CH_3)-$, $-CH=C(CH_3)-$, $-CH=C(i-C_3H_7)-$, $-CH_2-CH(i-C_3H_7)-$, $-CH=C(CH_3)-CH=C(CH_3)-$, $-C(C1)=C(CH_3)-$, $-CH_2-CH(CH_3)-CH_2-CH(CH_3)-$, $-CH_2-S-CH_2-$, $-S-CH_2-$, $-CH_2-S-CH(CH_3)-CH_2-$, $-S-CH(CH_3)-CH_2-$, $-CH_2-S-CH_2-CH_2-$, $-S-CH_2-CH_2-$, $-CH_2-S-CH_2-CH(CH_3)-$, $-S-CH_2-CH(CH_3)-$, $-CH_2-O-CH_2-$, $-O-CH_2-$, $-CH_2-O-CH(CH_3)-$, $-O-CH(CH_3)-$, $-CH_2-O-CH(CH_3)-CH_2-$, $-O-CH(CH_3)-CH_2-$, $-CH_2-O-(CH_2)_2-$, $-O-(CH_2)_2-$,

$—CH_2—O—CH_2—CH(CH_3)—$, $—O—CH_2—CH(CH_3)—$, $—CH_2—O—(CH_2)_3—$, $—O—(CH_2)_3—$.

$R^2$ in Formel I steht für unverzweigte oder verzweigte Alkylreste mit 1 bis 4 Kohlenstoffatomen, d. h. für Methyl, Ethyl, n-Propyl, i-Propyl, n-Butyl, sec.-Butyl, i-Butyl, tert.-Butyl.

Reste für $R^3$ in Formel I sind Propargyl, Alkyl mit 1 bis 3 Kohlenstoffatomen, Alkenyl mit 3 oder 4 C-Atomen oder Halogenalkenyl mit 3 oder 4 C-Atomen, das bis zu drei Halogensubstituenten enthalten kann, beispielsweise Methyl, Ethyl, n-Propyl, i-Propyl, n-Butyl, sec.-Butyl, i-Butyl, tert.-Butyl, Allyl, 1-Chlorprop-1-en-3-yl, 2-Chlorprop-1-en-3-yl, 1,3-Dichlorprop-1-en-3-yl, 1,1,2-Trichlorprop-1-en-3-yl.

Als Salze der Verbindungen der Formel I kommen beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze sowie Ammonium- und Phosphoniumsalze in Betracht.

Die Verbindungen der Formel I können durch Umsetzung von Verbindungen der Formel

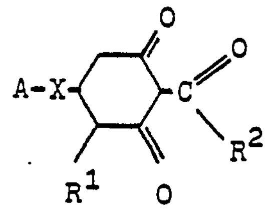

(II)

in der A, X, $R^1$ und $R^2$ die obengenannten Bedeutungen haben, mit Hydroxylaminderivaten $R^3O—NH_3Y$, in der $R^3$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung des pH-Bereichs erfolgt zweckmäßigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^3O—NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R^3O—NH_2$, in der $R^3$ die obengenannten Bedeutungen hat, in inerten Verdünnungsmitteln bei einer Temperatur zwischen 0 °C und dem Siedepunkt des Reaktionsgemisches, insbesondere zwischen 15 und 70 °C, erhalten werden. Gegebenenfalls kann das Hydroxylamin als wäßrige Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, z. B. die Mangan-, Kupfer-, Zink-, Eisen-, Calcium-, Magnesium- und Bariumsalze können aus den Natriumsalzen durch Reaktion mit den entsprechenden Metallchloriden in wäßriger Lösung hergestellt werden. Ammonium- und Phosphoniumsalze können durch Umsetzung von Verbindungen der Formel I mit Ammonium- oder Phosphoniumhydroxiden gegebenenfalls in wäßriger Lösung hergestellt werden.

Die Verbindungen der Formel II können aus Cyclohexan-1,3-dionen der Formel III, die auch in den tautomeren Formeln IIIa und IIIb vorliegen können,

(Siehe Formeln Seite 4 f.)

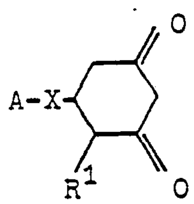

(III)

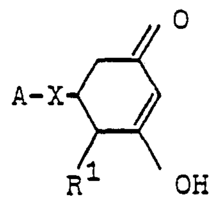

(IIIa)

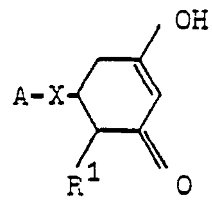

(IIIb)

nach literaturbekannten Methoden (Tetrahedron Letters, 29, 2 491 (1975)) hergestellt werden.

Es ist auch möglich, Verbindungen der Formel II über die Zwischenstufe der Enolester, die bei der Umsetzung von Verbindungen der Formel II eventuell als Isomerengemische anfallen und in Gegenwart von Imidazol- oder Pyridinderivaten umgelagert werden (JP-OS-79/063052), herzustellen.

Zu den Verbindungen der Formel III gelangt man nach literaturbekannten Verfahren, wie dies aus folgendem Schema hervorgeht :

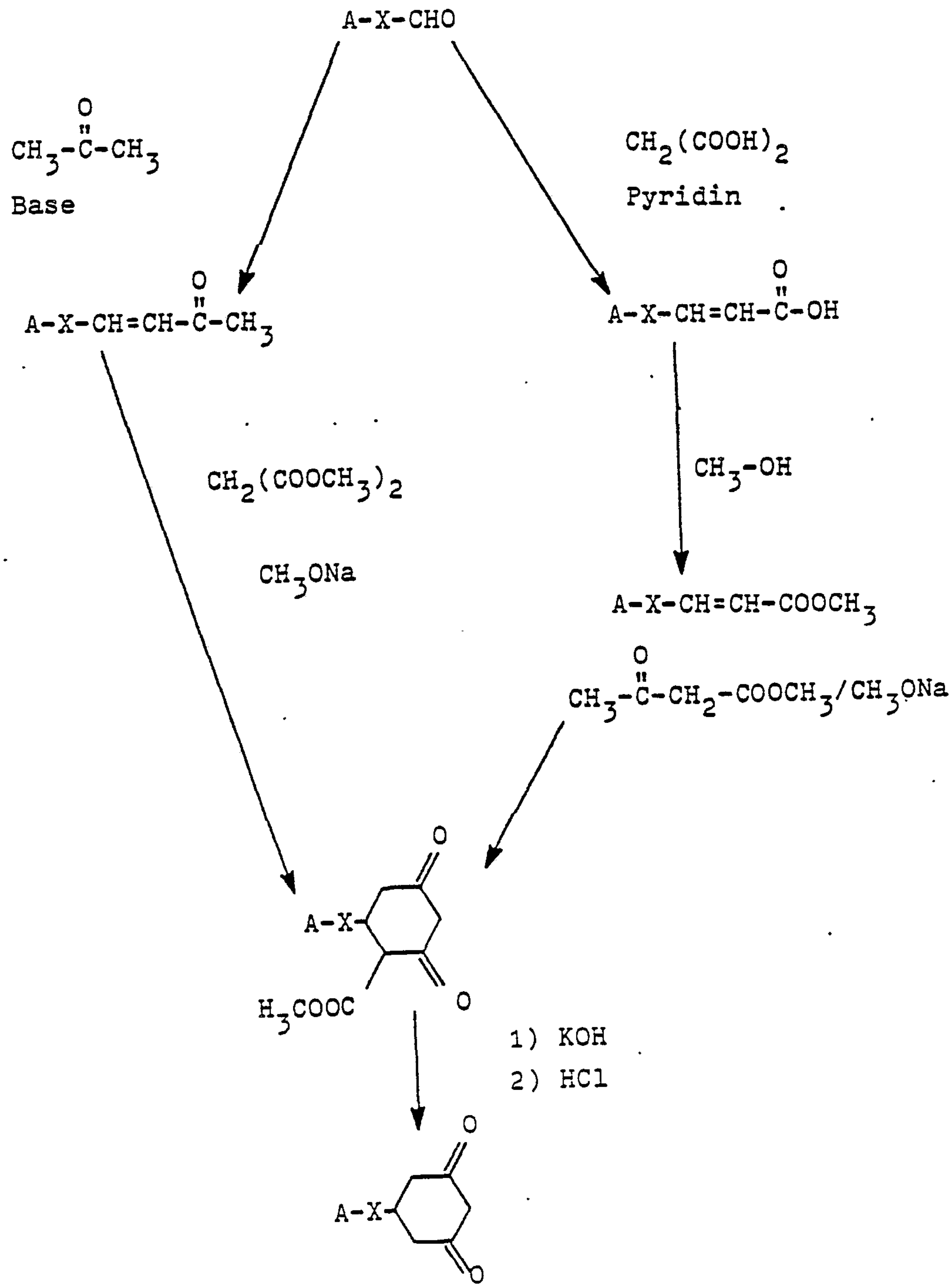

Die Aldehyde der Formel A—X—CHO sind ebenfalls nach literaturbekannten Methoden, z. B. durch Reduktion von entsprechenden Estern und Nitrilen, Oxidation von Alkoholen, Spaltung von Acetalen, Addition an α,β-ungesättigte Aldehyde zugänglich.

(Siehe Tabelle Seite 6 ff.)

Die Cyclohexandionderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Die Applikation kann im Vorauflauf- oder im Nachauflaufverfahren erfolgen. Vorzugsweise werden die neuen Wirkstoffe bzw. diese enthaltende Mittel nach dem Auflaufen der unerwünschten Pflanzen ausgebracht. Sie sind Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,05 bis 5 kg/ha, vorzugsweise 0,1 bis 2,0 kg/ha.

Die Wirkung der Cyclohexan-1,3-dionderivate der Formel I auf das Wachstum von erwünschten und unerwünschten Pflanzen läßt sich durch Gewächshausversuche zeigen :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender

Beispielsweise werden die folgenden Verbindungen genannt.

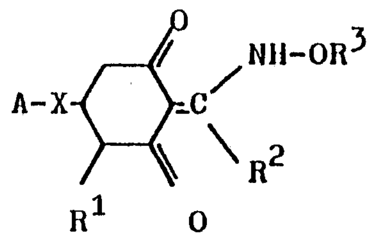

| Verbindung Nr. | A | X | $R^1$ | $R^2$ | $R^3$ | Physikal. Daten |
|---|---|---|---|---|---|---|
| 1 | Pyrid-3-yl | $-CH=C(CH_3)-$ | H | n-Propyl | " | $n_D^{27} = 1,5706$ |
| 2 | " | " | H | " | Ethyl | $n_D^{27} = 1,5662$ |
| 3 | " | " | H | " | Propargyl | $n_D^{27} = 1,5780$ |
| 4 | " | " | H | " | $-CH_2-CH=CHCl$ | |
| 5 | " | $-CH=C(CH_3)-CH=C(CH_3)-$ | H | " | Ethyl | $n_D^{36} = 1,5743$ |
| 6 | Pyrid-3-yl | $-CH=C(CH_3)-CH=C(CH_3)-$ | H | n-Propyl | Allyl | $n_D^{36} = 1,5725$ |
| 7 | Tetrahydropyran-3-yl | $-CH=C(CH_3)-$ | $COOCH_3$ | n-Propyl | Allyl | |
| 8 | " | " | $COCCH_3$ | " | Ethyl | |

(Fortsetzung)

| Verbindung Nr. | A | X | $R^1$ | $R^2$ | $R^3$ | Physikal. Daten |
|---|---|---|---|---|---|---|
| 9 | Tetrahydropyran-3-yl | $-CH=C(CH_3)-$ | $COOCH_3$ | n-Propyl | Ethyl | $n_D^{26} = 1,523$ |
| 10 | " | " | H | " | Allyl | $n_D^{26} = 1,529$ |
| 11 | " | " | H | " | $-CH_2-CH=CHCl$ | |
| 12 | 5,6-Dihydro-2H-pyran-3-yl | " | H | " | Ethyl | |
| 13 | " | " | H | " | Allyl | |
| 14 | Tetrahydropyran--2-yl | $-CH_2-O-CH(CH_3)-$ | H | " | " | |
| 15 | " | " | H | " | Ethyl | |
| 16 | Pyrid-2-yl | $-S-CH(CH_3)-CH_2-$ | H | n-Propyl | Ethyl | $n_D^{30} = 1,5562$ |
| 17 | " | " | H | " | Allyl | |

Düsen gespritzt. Die Aufwandmengen betragen 3,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Die Abdeckung bewirkt ein gleichmäßiges Keimen der Testpflanzen, sofern dies nicht durch die Wirkstoffe beeinträchtigt wird.

Für die Nachauflaufbehandlung zieht man die Testpflanzen je nach Wuchsform erst bis zu einer Wuchshöhe von 3 bis 15 cm an und behandelt sie danach. Die für die Nachauflaufanwendung benutzten Sojapflanzen sowie die Buschbohnen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff. Sie betragen 0,125 bzw. 0,25 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Alopecurus myosuroides (Ackerfuchsschwanz), Avena fatua (Flughafer), Beta vulgaris (Zuckerrübe), Echinochloa crus-galli (Hühnerhirse), Glycine max. (Soja), Gossypium hirsutum (Baumwolle), Lolium multiflorum (Ital. Raygras), Setaria faberii (Borstenhirse), Sorghum halepense (Sudangras), Triticum aestivum (Weizen), Setaria italica (Kolbenhirse).

Bei Vorauflaufanwendung zeigt beispielsweise die Verbindung 1 mit 3,0 kg Wirkstoff/ha eine sehr gute herbizide Wirkung gegen Grasarten.

Bei Nachauflaufanwendung bekämpft beispielsweise Verbindung Nr. 2 mit 0,25 kg Wirkstoff/ha grasartige unerwünschte Pflanzen, ohne die breitblättrigen Kulturpflanzen zu schädigen.

In Anbetracht der Verträglichkeit und der Vielseitigkeit der Applikationsmethoden können die erfindungsgemäßen Verbindungen noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Kübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Daucus carota | Möhre |
| Elaeis guineensis | Ölpalme |
| Fragaria vesca | Erdbeere |
| Glycine max | Sojabohne |
| Gossypium hirsutum (Gossypium arboreum<br>Gossypium herbaceumGossypium vitifolium) | Baumwolle |
| Helianthus annuus | Sonnenblume |
| Helianthus tuberosus | Topinambur |

| Botanischer Name | Deutscher Name |
|---|---|
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus duscis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |

Zur Verbreiterung des Wirkungsspektrums und zur Erzielung synergistischer Effekte können die Cyclohexan-1,3-dionderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. Cyclohexan-1,3-dion-derivate der Formel

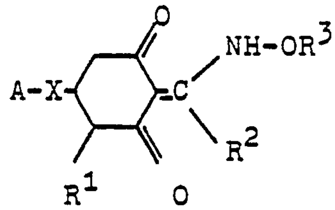

(I)

in der

A einen Pyridyl-, Tetrahydropyranyl- oder Dihydropyranyl rest, der gegebenenfalls durch $C_1$-$C_4$-Alkyl, $C_1$-$C_4$-Alkoxy oder Phenyl substituiert sein kann,

X eine Alkylenkette mit bis zu 5 C-Atomen oder eine Alkenylenkette mit bis zu 5 C-Atomen und bis zu 2 Doppelbindungen, die gegebenenfalls ein Schwefel- oder Sauerstoffatom oder eine Sulfinyl- oder Sulfonylgruppe enthalten und durch 2 Methylgruppen und Chlor substituiert sein können,
mit der Maßgabe, daß X keine reine gesättigte Kohlenstoffkette ist, wenn A ein nichtaromatischer heterocyclischer Rest mit maximal einer Doppelbindung und 1 Sauerstoffatom ist,

$R^1$ Wasserstoff, Methoxycarbonyl, Ethoxycarbonyl, Methyl oder Cyano,

$R^2$ Alkyl mit 1 bis 4 C-Atomen und

$R^3$ Alkyl mit 1 bis 3 C-Atomen, Alkenyl mit 3 oder 4 C-Atomen, Halogenalkenyl mit 3 oder 4 C-Atomen und 1 bis 3 Halogensubstituenten oder Propargyl bedeuten,
und Salze dieser Verbindungen.

2. Verfahren zur Herstellung eines Cyclohexan-1,3-dionderivates der Formel I gemäß Anspruch 1, dadurch gekennzeichnet, daß man eine Verbindung der Formel

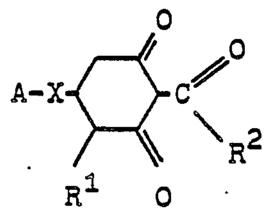

(II)

in der A, X, $R^1$ und $R^2$ die im Anspruch 1 genannten Bedeutungen haben,

a) mit einer Ammoniumverbindung der Formel $R^3O—NH_3Y$, in der $R^3$ die im Anspruch 1 genannten Bedeutungen hat und Y ein Anion bedeutet, in einem inerten Verdünnungsmittel gegebenenfalls in Gegenwart von Wasser bei einer Temperatur zwischen 0 und 80 °C in Gegenwart einer Base oder

b) mit einem gegebenenfalls in wäßriger Lösung vorliegenden Hydroxylamin der Formel $R^3O—NH_2$, in der $R^3$ die im Anspruch 1 genannten Bedeutungen hat, in einem inerten Lösungsmittel umsetzt.

3. Herbizid, enthaltend ein Cyclohexan-1,3-dionderivat der Formel I gemäß Anspruch 1.

4. Herbizid, enthaltend inerte Zusatzstoffe und ein Cyclohexan-1,3-dion-derivat der Formel I gemäß Anspruch 1.

5. Herbizid, enthaltend inerte Zusatzstoffe und 0,1 bis 95 Gew. % eines Cyclohexan-1,3-dionderivats der Formel I gemäß Anspruch 1.

6. Verfahren zur Bekämpfung unerwünschten Pflanzenwachstums, dadurch gekennzeichnet, daß man die unerwünschten Pflanzen und/oder die von unerwünschtem Pflanzenwachstum freizuhaltende Fläche mit einer herbizid wirksamen Menge eines Cyclohexan-1,3-dionderivates der Formel I gemäß Anspruch 1 behandelt.

**Claims**

1. A cyclohexane-1,3-dione derivative of the formula

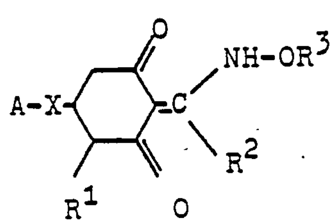

(I)

where

A is pyridyl, tetrahydropyranyl or dihydropyranyl optionally substituted by $C_1$-$C_4$-alkyl, $C_1$-$C_4$-alkoxy or phenyl,

X is an alkylene chain of up to 5 carbon atoms or an alkenylene chain containing up to 5 carbon atoms and up to 2 double bonds, which may or may not contain a sulfur or oxygen atom or a sulfinyl or sulfonyl group and is optionally substituted by 2 methyl groups and chlorine,
with the proviso that X is not a pure saturated hydrocarbon chain if A is a non-aromatic heterocyclic radical containing not more than one double bond and 1 oxygen atom,

$R^1$ is hydrogen, methoxycarbonyl, ethoxycarbonyl, methyl or cyano,

$R^2$ is alkyl of 1 to 4 carbon atoms, and

$R^3$ is alkyl of 1 to 3 carbon atoms, alkenyl of 3 or 4 carbon atoms, haloalkenyl containing 3 or 4 carbon atoms and 1 to 3 halogen substituents, or propargyl,
or a salt thereof.

2. A process for the preparation of a cyclohexane-1,3-dione derivative of the formula I as claimed in claim 1, wherein a compound of the formula

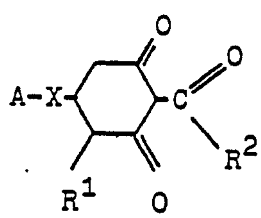

(II)

where A, X, $R^1$ and $R^2$ have the meanings given in claim 1, is reacted

a) with an ammonium compound of the formula $R^3O—NH_3Y$, where $R^3$ has the meanings given in claim 1, and Y is an anion, in an inert diluent in the presence or absence of water and in the presence of a base at a temperature of from 0 to 80 °C, or

b) with a hydroxylamine which may be present in the form of an aqueous solution and is of the formula $R^3O—NH_2$, where $R^3$ has the meanings given in claim 1, in an inert solvent.

3. A herbicide containing a cyclohexane-1,3-dione derivative of the formula I as claimed in claim 1.

4. A herbicide containing inert additives and a cyclohexane-1-3-dione derivative of the formula I as claimed in claim 1.

5. A herbicide containing inert additives and from 0.1 to 95 wt % of a cyclohexane-1,3-dione derivative of the formula I as claimed in claim 1.

6. A process for combating the growth of unwanted plants, wherein the unwanted plants and/or the area to be kept free from unwanted plant growth are treated with a herbicidally effective amount of a cyclohexane-1,3-dione derivative of the formula I as claimed in claim 1.

**Revendications**

1. Dérivés de cyclohexane-1,3-dione de formule

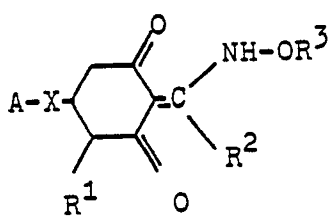

(I)

dans laquelle

A représente un radical pyridyle, tétrahydropyranyle ou dihydropyranyle, qui peut être éventuellement substitué par un groupement alkyle en $C_1$-$C_4$, alcoxy en $C_1$-$C_4$ ou phényle,

X représente une chaîne alkylène pouvant contenir jusqu'à 5 atomes de C ou une chaîne alcénylène contenant jusqu'à 5 atomes de C et jusqu'à 2 doubles liaisons, qui contient éventuellement un atome de soufre ou d'oxygène ou un groupement sulfinyle ou sulfonyle et peut être substituée par 2 groupements méthyle et atomes de chlore,
avec cette condition que X ne soit pas une chaîne carbonée pure saturée lorsque A est un radical hétérocyclique non aromatique comportant au maximum une double liaison et 1 atome d'oxygène,

$R^1$ représente un atome d'hydrogène, un groupement méthoxycarbonyle, éthoxycarbonyle, méthyle ou cyano,

$R^2$ représente un radical alkyle à 1-4 atomes de C et

$R^3$ représente un radical alkyle à 1-3 atomes de C, alcényle à 3 ou 4 atomes de C, halogéno-alcenyle à 3 ou 4 atomes de C et à 1-3 substituants halogènes, ou propargyle,

et sels de ces composés.

2. Procédé de préparation d'un dérivé de cyclohexane-1,3-dione de formule I selon la revendication 1, caractérisé en ce qu'on fait réagir dans un solvant inerte un composé de formule

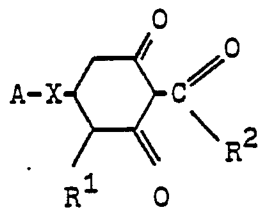

(II)

dans laquelle A, X, $R^1$ et $R^2$ ont les significations données dans la revendication 1,

a) avec un composé d'ammonium de formule $R^3O—NH_3Y$, dans laquelle $R^3$ a les significations données dans la revendication 1 et Y représente un anion, dans un diluant inerte, le cas échéant en présence d'eau, à une température comprise entre 0 et 80 °C, en présence d'une base ou

b) avec une hydroxylamine de formule $R^3O—NH_2$, dans laquelle $R^3$ a les significations données dans la revendication 1, se présentant éventuellement en solution aqueuse.

3. Herbicide contenant un dérivé de cyclohexane-1,3-dione de formule I selon la revendication 1.

4. Herbicide contenant des additifs inertes et un dérivé de cyclohexane-1,3-dione de formule I selon la revendication 1.

5. Herbicide contenant des additifs inertes et 0,1 à 95 % en poids d'un dérivé de cyclohexane-1,3-dione de formule I selon la revendication 1.

6. Procédé de lutte contre la croissance de plantes indésirables, caractérisé en ce qu'on traite les plantes indésirables et/ou les surfaces à protéger contre la croissance de plantes indésirables par une quantité, suffisante pour avoir un pouvoir herbicide, d'un dérivé de cyclohexane-1,3-dione de formule I selon la revendication 1.